# EUROPÄISCHE PATENTSCHRIFT

(11) **EP 2 697 366 B1**
(45) Veröffentlichungstag und Bekanntmachung des Hinweises auf die Patenterteilung: **05.09.2018**
(21) Anmeldenummer: 12713961.6
(22) Anmeldetag: 05.04.2012
(51) Int. Cl.: C12N 5/0775, A61K 35/28, A61K 38/58, A61K 35/30

(54) **BIVALIRUDIN ZUR KONSERVIERUNG DER ZELLULÄREN FUNKTIONALITÄT IN DER KLINISCHEN ZELLTHERAPIE**
BIVALIRUDIN FOR THE PRESERVATION OF CELLULAR FUNCTIONALITY IN CLINICAL CELL THERAPY
BIVALIRUDINE POUR LA CONSERVATION DE LA FONCTIONNALITÉ CELLULAIRE DANS LA THÉRAPIE CELLULAIRE CLINIQUE

(30) Priorität: 15.04.2011 DE 102011017208
(43) Veröffentlichungstag der Anmeldung: 19.02.2014
(73) Patentinhaber: Johann Wolfgang Goethe-Universität, Frankfurt am Main, 60323 Frankfurt am Main (DE)
(72) Erfinder: SEEGER, Florian, 64347 Griesheim (DE); ASSMUS, Birgit, 61449 Steinbach (DE); DIMMELER, Stefanie, 60594 Frankfurt (DE); ZEIHER, Andreas, 60549 Frankfurt (DE)
(74) Vertreter: Krauss, Jan
(86) Internationale Anmeldenummer: PCT/EP2012/056292
(87) Internationale Veröffentlichungsnummer: WO 2012/139970

(56) Entgegenhaltungen:
- EP-A2- 1 771 551
- WO-A2-03/064687
- WO-A2-2011/030332
- VOLKER SCHÄCHINGER ET AL: "Intracoronary Bone Marrow-Derived Progenitor Cells in Acute Myocardial Infarction", NEW ENGLAND JOURNAL OF MEDICINE, Bd. 355, Nr. 12, 21. September 2006 (2006-09-21), Seiten 1210-1221, XP55031575, ISSN: 0028-4793, DOI: 10.1056/NEJMoa060186
- SEEGER FLORIAN ET AL: "v Heparin Disrupts the CXCR4/SDF-1 Axis and Impairs the Functional Capacity of Bone Marrow-Derived Mononuclear Cells (BMC) Used for Cardiovascular Repair: Implications for the Heterogeneous Results of Clinical Cell Therapy Trials in AMI", CIRCULATION, Bd. 124, Nr. 21, Suppl. S, 22. November 2011 (2011-11-22), Seite A12098, XP008153210, SCIENTIFIC SESSIONS OF THE AMERICAN-HEART-ASSOCIATION/RESUSCITATION SCIENCE SYMPOSIUM; ORLANDO, FL, USA; NOVEMBER 12 -16, 2011
- A. Hirsch ET AL: "Intracoronary infusion of mononuclear cells from bone marrow or peripheral blood compared with standard therapy in patients after acute myocardial infarction treated by primary percutaneous coronary intervention: results of the randomized controlled HEBE trial", European Heart Journal, vol. 32, no. 14, 10 December 2010 (2010-12-10), pages 1736-1747, XP55272383, GB ISSN: 0195-668X, DOI: 10.1093/eurheartj/ehq449

## Beschreibung

Die vorliegende Erfindung betrifft pharmazeutische Zusammensetzungen zur Zelltherapie, umfassend zur Stammzelltherapie geeignete nicht-embryonale Stammzellen und als einzige Gerinnungs-inhibierende Komponente Bivalirudin, wobei die nicht-embryonalen Stammzellen Knochenmark-abgeleitete Zellen (BMC) sind. Die vorliegende Erfindung betrifft weiterhin die entsprechende Verwendung dieses Thrombinhemmers insbesondere im Rahmen der Zelltherapie für kardiovaskuläre Erkrankungen.

### Hintergrund der Erfindung

Für kein anderes Antikoagulanz außer Heparin besteht eine Zulassung bei kardiochirurgischen Eingriffen. Als problematisch erweist sich deshalb die Notwendigkeit einer Herzoperation bei Patienten mit einer Heparininduzierten Thrombopenie (HIT); einer Erkrankung bei der Antikörper gegen einen Komplex aus Heparin und Plättchenfaktor 4 gebildet werden und die mit massiven thromboembolischen Komplikationen vergesellschaftet ist. Obwohl mit zahlreichen Substanzen der Versuch unternommen wurde, bei Patienten mit HIT eine sichere Antikoagulation bei kardiochirurgischen Eingriffen vorzunehmen, waren diese Versuche zumeist mit massiven Blutungskomplikationen verbunden da für keine der alternativen Substanzen ein Antidot zur Verfügung steht.

Die peptidischen Thrombininhibitoren wurden ausgehend von Hirudin entwickelt, einem gerinnungshemmenden Polypeptid mit 65 Aminosäuren aus dem Blutegel, *Hirudo medicinalis.* Sie müssen parenteral verabreicht werden. Bivalirudin, ein Peptid mit 20 Aminosäurensequenzen, ist ein direkter, bivalenter reversibler Thrombinhemmer mit einer kurzen Eliminationshalbwertszeit von ca. 25 Minuten. Bivalirudin hemmt das Thrombin durch Bindung an das aktive Zentrum und die Exosite 1 Region. Diese Hemmung ist reversibel, da das Thrombin das Bivalirudin spaltet und die beiden Fragmente eine geringe Affinität zum Thrombin haben

In zwei großen, multizentrischen, randomisierten, doppelblinden und placebokontrollierten Studien führte Bivalirudin zu einer hochsignifikanten Reduktion schwerer Blutungskomplikationen. Die antithrombotische Aktivität im Hinblick auf typische Ischämieendpunkte war dabei der Standardtherapie mit Heparin vergleichbar. Die einfache Handhabung und das deutlich geringere Blutungsrisiko bei gleich guter Effektivität im Hinblick auf ischämische Endpunkte sprechen für den Einsatz von Bivalirudin im Rahmen von Koronarinterventionen.

Bisherige Studien und die entsprechende Metaanalyse lassen vermuten, dass aus dem Knochenmark gewonnene (BMC) einen positiven Einfluss bei der Neovaskularisierung und auf die Herzfunktionen nach akutem Myokardinfarkt (Martin-Rendon et al., Lipinski et al., Schächinger et al.), bei chronischer Herzschwäche (Assmus et al.), bei refraktärer Angina (Losordo et al.) sowie bei peripherer arterieller Verschlusskrankheit (Walter et al.) haben. Sowohl die Auswahl der Patienten, die Zahl und insbesondere die funktionelle Aktivität (Britten et al., Walter et al.) der infundierten Zellen scheinen für den Erfolg der Zelltherapie wesentlich zu sein.

Die funktionelle Aktivität von BMC wird herkömmlich auf Basis ihrer migratorischen Kapazität bestimmt, mit der das "Recruitment" und die Fähigkeit zum Einwandern ("Homing") in das Zielgewebe bestimmt werden kann, was die Grundlage für die vorteilhaften Effekte auf die Neovaskularisierung und funktionelle Regeneration darstellt.

Kürzlich wurde gezeigt, dass unterschiedliche Protokolle zur Isolierung von Zellen für die Zelltherapie einen wesentlichen Einfluss auf die funktionelle Aktivität von BMC haben (Seeger et al. 2007).

Heparin wird bei der Infusion von Stammzellen zur Behandlung von Myokardinfarkten eingesetzt (Hirsch et al: "Intracoronary infusion of mononuclear cells from bone marrow or peripheral blood compared with standard therapy in patients after acute myocardial infarction treated by primary percutaneous coronary intervention: results of the randomized controlled HEBE trial", European Heart Journal, GB, (20101210), Band 32, Nr. 14, Seiten 1736 - 1747.

Während der Präparation, Lagerung und Verabreichung an Patienten ist eine Antikoagulation des Zellprodukts unverzichtbar, um ein Gerinnen oder Verklumpen des Zellprodukts zu verhindern und das Risiko einer mikrovaskulären Embolie während der intra-arteriellen Verabreichung von Zellen zu eliminieren.

Es ist daher die Aufgabe der vorliegenden Erfindung, verbesserte Zusammensetzungen zur Verwendung bei der Isolierung, Aufbewahrung und Applikation von Zellen für die klinische Zelltherapie und insbesondere BMC zur Verfügung zu stellen.

Gemäß einem ersten Aspekt der vorliegenden Erfindung wird diese Aufgabe gelöst durch eine pharmazeutische Zusammensetzung zur Zelltherapie, umfassend zur Stammzelltherapie geeignete nicht-embryonale Stammzellen und als einzige Gerinnungs-inhibierende Komponente Bivalirudin, wobei die nicht-embryonalen Stammzellen Knochenmark-abgeleitete Zellen (BMC) sind..

Die Erfindung basiert auf dem Ersatz von Heparin als herkömmlicher Bestandteil von pharmazeutischen Zusammensetzungen zur Zelltherapie, da Heparin überraschend als diejenige Komponente identifiziert wurde, die die migratorische Kapazität von Stammzellen, wie zum Beispiel aus dem Knochenmark-gewonnene mononukleäre Zellen (BMC) durch Beeinträchtigung des SDF-1 / CXCR4-Signalwegs beeinträchtigt.

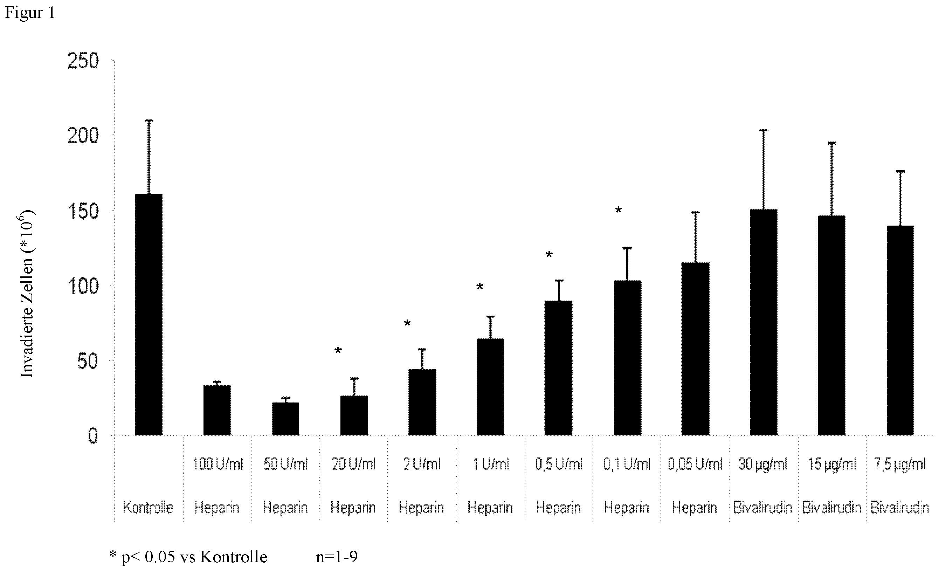

Das Chemokin SDF-1 (Stromal cell-derived factor-1) und sein spezifischer Rezeptor, Chemokinrezeptor 4 (CXCR4), spielen eine entscheidende Rolle bei der postnatalen Neovaskularisierung. SDF-1 wird in Antwort auf Hypoxie freigesetzt und rekrutiert CXCR4 exprimierende vaskulogene Zellen zu Stellen der Ischämie (Ceradini et al.; Grunewald et al.; Salcedo et al.; Walter et al.; Zhang et al.). Kürzlich wurde gezeigt, dass unfraktioniertes Heparin sowie niedermolekulares Heparin in klinisch relevanten Konzentrationen die CXCR4 Rezeptorbindung und die Antwort auf das Chemoattraktant SDF-1 in Krebszellen (Harvey et al., Ma et al.) inhibiert. Daher könnte die Beeinträchtigung des SDF-1/CXCR4-Signalweges durch eine Heparinbehandlung die funktionelle BMC-Aktivität beeinflussen. In der Tat zeigten Experimente eine deutliche Dosis-abhängige Reduktion der Invasionskapazität von Heparinbehandelten BMC (jeweils 20 U/l Heparin 16±4 % Kontrolle, 2 U/l Heparin 27±7 % Kontrolle p<0.05) (siehe Abb. 1). Ähnlich könnten andere Antikoagulantien die SDF-1/CXCR-4 Interaktion beeinträchtigten.

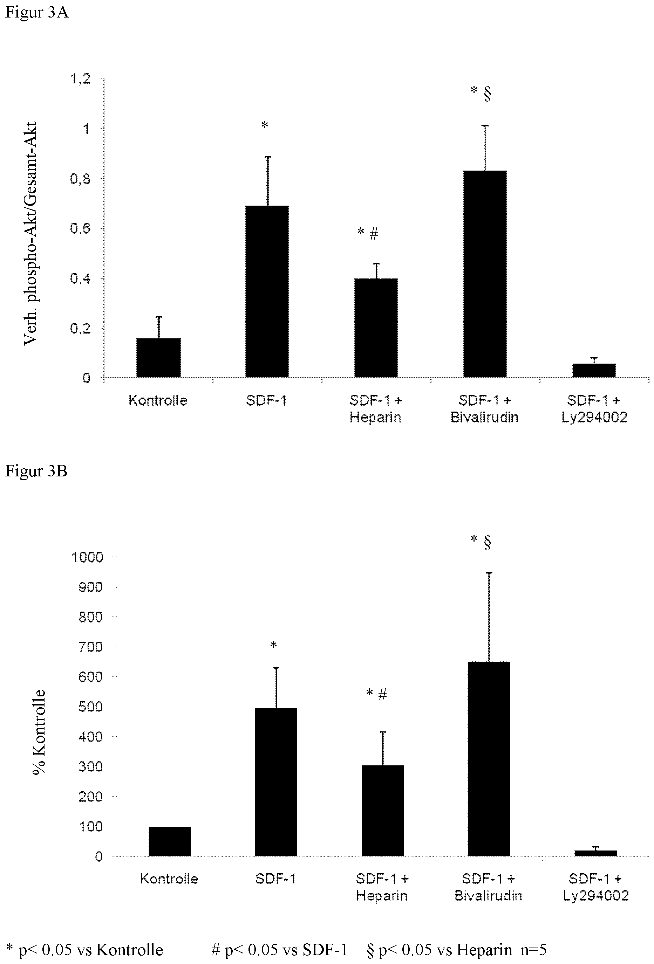

Die Erfinder belegen, dass unter Verwendung von Bivalirudin als klinisch für die Gerinnungshemmung zugelassener Thrombinhemmer die SDF-1-vermittelte Interaktion mit dem CXCR-4-Receptor nicht beeinträchtigt ist, wie durch Migrationstests (Abb. 1) und die Analyse der Akt-Phosphorylierung als dem stromabwärtigen Effektorsignal der CXCR-4 Aktivierung durch SDF-1 (Abb. 3) gemessen wurde. Aller Wahrscheinlichkeit nach werden auch andere nicht Hirudin-basierte Antikoagulantien die Funktionalität der verabreichten Zellen für die Zelltherapie bei kardiovaskulärer Erkrankung nicht beeinträchtigen.

Bevorzugt werden Zellen verabreicht, die im Rahmen der Zelltherapie bei kardiovaskulären Erkrankungen einen positiven Einfluss bei der Neovaskularisierung, der Herzfunktion nach akutem Myokardinfarkt (Martin-Rendon et al., Lipinski et al., Schächinger et al.), bei chronischer Herzschwäche (Assmus et al.), bei refraktärer Angina (Losordo et al.) sowie bei peripherer arterieller Verschlusskrankheit (Walter et al.) haben.

Bevorzugt ist eine erfindungsgemäße pharmazeutische Zusammensetzung, wobei die nicht-embryonalen Stammzellen Knochenmark-abgeleitete Zellen (BMC) sind.

Ein weiterer Aspekt der vorliegenden Erfindung betrifft dann eine pharmazeutische Zusammensetzung der vorliegenden Erfindung zur Verwendung zur Behandlung von Erkrankungen. Weiter bevorzugt ist dann eine erfindungsgemäße pharmazeutische Zusammensetzung zur Verwendung bei der Behandlung von kardiovaskulären Erkrankungen, zum Beispiel Myokardinfarkt, chronische Herzschwäche, refraktärer Angina und peripherer arterieller Verschlusskrankheit.

Die entsprechende Art der Verabreichung, insbesondere bei der Zelltherapie mittels aus dem Knochenmark gewonnenen mononuklären Zellen (BMC), ist aus dem Stand der Technik bestens bekannt (siehe z.B. Kang S, Yang YJ, Li CJ, Gao RL. Effects of intracoronary autologous bone marrow cells on left ventricular function in acute myocardial infarction: a systematic review and meta-analysis for randomized controlled trials. Coron Artery Dis. 2008 Aug;19(5):327-35 und darin zitierte Publikationen).

Ein weiterer Aspekt der vorliegenden Erfindung betrifft dann ein Verfahren zur Herstellung einer zur Stammzelltherapie geeigneten pharmazeutischen Zusammensetzung, umfassend das Mischen von isolierten Knochenmark-abgeleitete Zellen (BMC) mit mindestens Bivalirudin als einzige Thrombin-inhibierende Komponente. Das Verfahren umfasst weiterhin die optionale Hinzufügung von weiteren im Rahmen der Stammzelltherapie geeigneten Zusatz- und Hilfsstoffen, wie zum Beispiel Ficoll, X-vivo medium, Plasma oder Kochsalzlösung. Alle Bestandteile sind dabei frei von Gerinnungs-inhibierenden Komponenten, die mit dem SDF-1 / CXCR4-Signalweg interferieren, wie etwa Heparin.

Wie bereits angegeben, kann die funktionelle Aktivität der entsprechend hergestellten/präparierten Stammzellen durch Migrationstests (Abb. 1) und die Analyse der Akt-Phosphorylierung als dem stromabwärtigen Effektorsignal der CXCR-4 Aktivierung durch SDF-1 (Abb. 3) gemessen und somit überprüft werden. Geeignet sind auch andere Tests, wie zum Beispiel Vitalitätsfärbungen und die Fähigkeit der Zellen zur Koloniebildung (cfu). Alle diese Tests sind aus dem Stand der Technik bekannt.

Weiter bevorzugt ist ein erfindungsgemäßes Verfahren, das weiterhin eine geeignete Lagerung der pharmazeutischen Zusammensetzung umfasst. Diese kann bevorzugt in Medium über Nacht oder auf geeignete Weise gekühlt oder eingefroren durchgeführt werden.

Ein weiterer Aspekt der vorliegenden Erfindung betrifft dann die *in vitro* Verwendung von Bivalirudin als einzige Thrombin-inhibierender Komponente bei der Präparation und/oder Lagerung von zur Zelltherapie geeigneten BMC.

Ein noch weiterer Aspekt der vorliegenden Erfindung betrifft dann die *in vitro* Verwendung von Bivalirudin, als Ersatz für Heparin bei der Präparation und Lagerung von zur Stammzelltherapie geeigneten BMC.

Ein noch weiterer Aspekt der vorliegenden Offenbarung betrifft dann ein Verfahren zur Behandlung von kardiovaskulären Erkrankungen, zum Beispiel Myokardinfarkt, chronischer Herzschwäche, refraktärer Angina und peripherer arterieller Verschlusskrankheit, umfassend die Verabreichung einer pharmazeutischen Zusammensetzung gemäß der vorliegenden Erfindung an den Patienten. Es werden dabei Zellen verabreicht, die im Rahmen der Zelltherapie bei kardiovaskulären Erkrankungen einen positiven Einfluss haben. Entsprechende Verabreichungsprotokolle sind aus dem Stand der Technik und der hier beschriebenen Literatur bekannt. Der Patient ist ein Säuger, bevorzugterweise eine Maus, Ratte, Affe oder Mensch, weiter bevorzugt ein Mensch.

Die Erfindung soll nun im Weiteren durch die beigefügten Beispiele beschrieben werden, ohne jedoch darauf beschränkt zu sein.
**Figur 1** zeigt die SDF-1 induzierte Invasionskapazität von humanen BMC in Abhängigkeit der Inkubation mit verschiedenen Konzentrationen von Heparin oder Bivalirudin. * p<0,05 vs. Kontrolle, n=1-9.

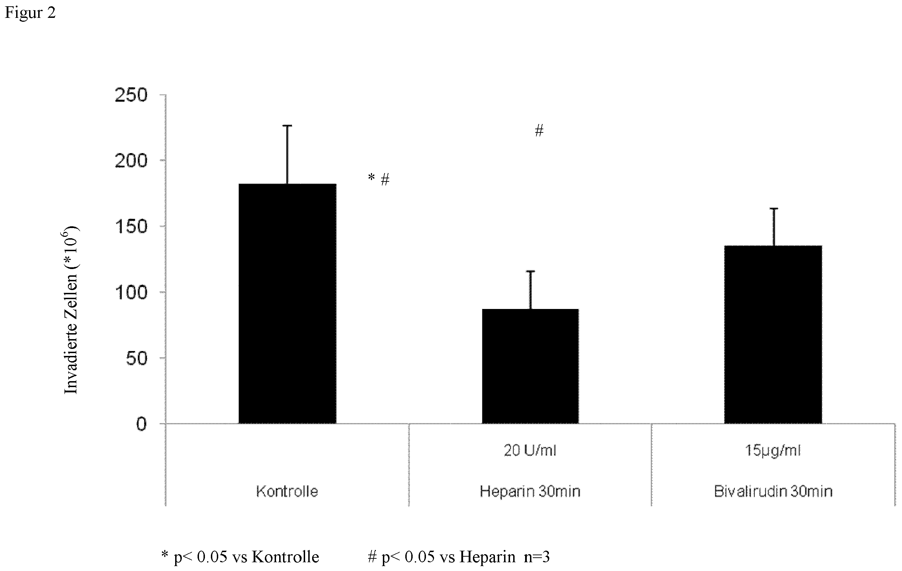

**Figur 2** zeigt die SDF-1 induzierte Invasionskapazität von humanen BMC in Abhängigkeit der Vorinkubation mit Heparin oder Bivalirudin, das nach 30 Minuten wieder herausgewaschen wurde. * p<0,05 vs. Kontrolle, # p<0,05 vs. Bivalirudin, n=3.
**Figur 3A** zeigt das Verhältnis zwischen phosphoryliertem und Gesamt-Akt von BMC nach SDF-1 Stimulation in Abhängigkeit der Vorinkubation mit Heparin (20U/ml) oder Bivalirudin (15mg/ml). * p<0,05 vs. Kontrolle, # p<0,05 vs. SDF-1, § p<0,05 vs. Heparin, n=5.
**Figur 3B** zeigt das Verhältnis zwischen phosphoryliertem und Gesamt-Akt von BMC nach SDF-1 Stimulation in Abhängigkeit der Vorinkubation mit Heparin (20 U/ml) oder Bivalirudin (15 µg/ml) relativ auf die unstimulierte Kontrolle bezogen. * p<0,05 vs. Kontrolle, # p<0,05 vs. SDF-1, § p<0,05 vs. Heparin, n=5.

Es konnte keine Induktion von Apoptose oder Nekrose bei den Zellen gefunden werden.

### Beispiele

### Isolierung und Behandlung der mononukleären Knochenmarkzellen (BMC)

Die mononukleären Zellen wurden aus filtriertem und 1:5 mit PBS verdünntem Knochenmarkaspirat von gesunden, freiwilligen Spendern mittels Ficoll-Dichtezentrifugation gewonnen (Biochrom AG; 800xg ohne Bremse). Die mononukleären Zellen (BMC) wurden dreimal mit PBS gewaschen, gezählt und für die Versuche verwendet.

Zur Bestimmung der Invasionskapazität wurden die isolierten Knochenmarkzellen konzentrationsabhängig für 30 Minuten mit Heparin (100U/ml - 0,05 U/ml) oder Bivalirudin (Angiox®; 7,5-30 µg/ml) inkubiert. Für einen Teil der Versuche wurde Heparin/Bivalirudin nach der 30-minütigen Inkubationsphase im Brutschrank zweimal mit PBS ausgewaschen.

### Invasionassay

Zur Bestimmung der funktionellen Aktivität der Zellen wurde eine modifizierte, mit Matrigel gefüllte Boyden-Invasionskammer (BioCoat Matrigel Invasion Assay, 8µm Pore Size, Becton Dickinson) verwendet. 1x10⁶ BMC wurden in 250 µl x-vivo 15 Medium (Lonza) in die obere Kammer gefüllt und die Kammer in eine 24-Well Platte überführt, die 500 µl x-vivo 15 Medium mit 100 ng/ml SDF-1 (R&D) enthielt. Nach 24 Stunden Inkubation bei 37°C wurden die transmigrierten Zellen mittels Neubauer-Zählkammer ausgezählt. Alle Versuche wurden in Doppelwerten durchgeführt.

### Akt-ELISA

8x10⁶ BMC pro Ansatz wurden über Nacht bei 37°C mit Heparin (20 U/ml), Bivalirudin (15 µg/ml) oder PBS in 500µl x-vivo 10 Medium inkubiert. Nach 30-minütiger Vorstimulation mit Ly294002 (25 µM, Sigma) erfolgte eine SDF-1 Stimulation (2,5 Minuten, 100 ng/ml). Die Zellen wurden sofort mit eiskaltem PBS gewaschen und Protein nach Herstellerangaben des Akt-ELISA-Kits (Cell Signaling) isoliert. Nach entsprechender Verdünnung der Proben erfolgte die Inkubation in den ELISA-Platten nach Herstellerangaben über Nacht bei 4°C. Am Folgetag wurden, nach Wasch- und Detektionsschritten, die Werte für Gesamt-Akt und phosphoryliertes Akt mittels ELISA-Reader detektiert. Die Abbildungen zeigen das Verhältnis zwischen phosphoryliertem und Gesamt-Akt.

### Zitierte Literatur

Seeger, F. H., et al. Cell isolation procedures matter: a comparison of different isolation protocols of bone marrow mononuclear cells used for cell therapy in patients with acute myocardial infarction. Eur Heart J 28: 766-72, 2007
Seeger, F. H., et al. CXCR4-expression determines functional activity of bone marrow-derived mononuclear cells for therapeutic neovascularization in acute ischemia. ATVB 29: 1820-09, 2009
Walter, D. H., et al. Impaired CXCR4 Signaling Contributes to the Reduced Neovascularization Capacity of Endothelial Progenitor Cells From Patients With Coronary Artery Disease. Circ Res 97: 1142-51, 2005
Martin-Rendon E., et al. Autologous bone marrow stem cells to treat acute myocardial infarction: a systematic review. Eur Heart J. 29:1807-18, 2008
Lipinski M.J., et al. Impact of intracoronary cell therapy on left ventricular function in the setting of acute myocardial infarction: a collaborative systematic review and meta-analysis of controlled clinical trials. J Am Coll Cardiol. 18:1761-7, 2007
Schächinger V., et al. REPAIR-AMI Investigators. Intracoronary bone marrow-derived progenitor cells in acute myocardial infarction. N Engl J Med. 355:1210-21, 2006
Britten M.B., et al. Infarct remodeling after intracoronary progenitor cell treatment in patients with acute myocardial infarction (TOPCARE-AMI): mechanistic insights from serial contrast-enhanced magnetic resonance imaging. Circulation 2003 108:2212-8, 2003.
Ceradini, D. J., et al. Progenitor cell trafficking is regulated by hypoxic gradients through HIF-1 induction of SDF-1. Nat Med 10: 858-64, 2004
Grunewald, M., et al. VEGF-induced adult neovascularization: recruitment, retention, and role of accessory cells. Cell 124: 175-89, 2006
Salcedo, R., et al. Vascular endothelial growth factor and basic fibroblast growth factor induce expression of CXCR4 on human endothelial cells: In vivo neovascularization induced by stromal-derived factor-lalpha. Am J Pathol 154: 1125-35., 1999
Zhang, M., et al. SDF-1 expression by mesenchymal stem cells results in trophic support of cardiac myocytes after myocardial infarction. FASEB J 21: 3197-207, 2007
Harvey J.R., et al. Inhibition of CXCR4-mediated breast cancer metastasis: a potential role for heparinoids? Clin Cancer Res. 13:1562-70, 2007
Ma L., , et al. Modulating the interaction of CXCR4 and CXCL12 by low-molecular-weight heparin inhibits hepatic metastasis of colon cancer. Invest New Drugs. Nov 16. Epub ahead of print, 2010
Walter DH, et al.; for the PROVASA Investigators. Intraarterial Administration of Bone Marrow Mononuclear Cells in Patients With Critical Limb Ischemia: A Randomized-Start, Placebo-Controlled Pilot Trial (PROVASA). Circ Cardiovasc Interv.4(1):26-37; 2011
Assmus B, et al. Transcoronary transplantation of progenitor cells after myocardial infarction. N Engl J Med. 355(12):1222-32; 2006
Losordo DW, et al. Intramyocardial transplantation of autologous CD34+ stem cells for intractable angina: a phase I/IIa double-blind, randomized controlled trial. Circulation. 115(25):3165-72; 2007

## Patentansprüche

1. Pharmazeutische Zusammensetzung zur Zelltherapie, umfassend zur Stammzelltherapie geeignete nicht-embryonale Stammzellen und als einzige Gerinnungs-inhibierende Komponente Bivalirudin, wobei die nicht-embryonalen Stammzellen Knochenmark-abgeleitete Zellen (BMC) sind.

2. Pharmazeutische Zusammensetzung nach Anspruch 1 zur Verwendung zur Behandlung von Erkrankungen.

3. Pharmazeutische Zusammensetzung zur Verwendung nach Anspruch 2 zur Verwendung zur Behandlung von kardiovaskulären Erkrankungen.

4. Pharmazeutische Zusammensetzung zur Verwendung nach Anspruch 3, wobei die kardiovaskuläre Erkrankung ausgewählt wird aus Myokardinfarkt, chronischer Herzschwäche, refraktärer Angina und peripherer arterieller Verschlusskrankheit.

5. Pharmazeutische Zusammensetzung zur Verwendung nach einem der Ansprüche 2 bis 4, wobei die Verwendung die Verabreichung der pharmazeutischen Zusammensetzung an den Patienten umfasst.

6. Verfahren zur Herstellung einer zur Stammzelltherapie geeigneten pharmazeutischen Zusammensetzung, umfassend das Mischen von isolierten nicht-embryonalen Stammzellen, wobei die nicht-embryonalen Stammzellen Knochenmark-abgeleitete Zellen (BMC) sind, mit mindestens Bivalirudin als einzige Thrombin-inhibierende Komponente.

7. Verfahren nach Anspruch 6, weiterhin umfassend eine geeignete Lagerung der pharmazeutischen Zusammensetzung.

8. *In vitro* Verwendung von Bivalirudin als einzige Thrombin-inhibierender Komponente bei der Präparation und/oder Lagerung von zur Zelltherapie geeigneten BMC.

9. *In vitro* Verwendung von Bivalirudin, als Ersatz für Heparin bei der Präparation und Lagerung von zur Stammzelltherapie geeigneten BMC.

## Claims

1. Pharmaceutical composition for cell therapy, comprising non-embryonic stem cells suitable for stem cell therapy and bivalirudin as sole coagulation-inhibiting component, wherein the non-embryonic stem cells are bone marrow-derived cells (BMC).

2. Pharmaceutical composition according to claim 1, for use in the treatment of diseases.

3. Pharmaceutical composition according to claim 2, for use in the treatment of cardiovascular diseases.

4. Pharmaceutical composition for use according to claim 3, wherein the cardiovascular disease is selected from myocardial infarction, chronical heart failure, refractory angina and peripheral arterial occlusive disease.

5. Pharmaceutical composition for use according to any of claims 2 to 4, wherein the use comprises administration of the pharmaceutical composition to the patient.

6. Method for manufacturing a pharmaceutical composition suitable for stem cell therapy, comprising mixing isolated non-embryonic stem cells, wherein the non-embryonic stem cells are bone marrow-derived cells (BMC), with at least bivalirudin as sole thrombin-inhibiting component.

7. Method according to claim 6, further comprising appropriate storage of the pharmaceutical composition.

8. *In vitro* use of bivalirudin as sole thrombin-inhibiting component in the preparation and/or storage of BMC suitable for cell therapy.

9. *In vitro* use of bivalirudin as a substitute for heparin in the preparation and storage of BMC suitable for stem cell therapy.

## Revendications

1. Composition pharmaceutique pour la thérapie cellulaire, comprenant des cellules souches non embryonnaires appropriées pour la thérapie avec cellules souches et comme unique composant anti-coagulation de la bivalirudine, dans laquelle les cellules souches non embryonnaires sont des cellules dérivées de la moelle osseuse (BMC).

2. Composition pharmaceutique selon la revendication 1 destinée à être utilisée dans le traitement de maladies.

3. Composition pharmaceutique destinée à être utilisée selon la revendication 2, destinée à être utilisée dans le traitement de maladies cardiovasculaires.

4. Composition pharmaceutique destinée à être utilisée selon la revendication 3, dans laquelle la maladie cardiovasculaire est choisie parmi l'infarctus du myocarde, l'insuffisance cardiaque chronique, l'angine réfractaire et l'occlusion artérielle périphérique.

5. Composition pharmaceutique destinée à être utilisée selon l'une des revendications 2 à 4, dans laquelle l'utilisation comprend l'administration de la composition pharmaceutique à des patients.

6. Procédé de préparation d'une composition pharmaceutique appropriée pour la thérapie cellulaire, comprenant le mélange de cellules souches non embryonnaires isolées, dans lequel les cellules souches non embryonnaires sont des cellules dérivées de la moelle osseuse (BMC), avec au moins de la bivalirudine comme unique composant inhibiteur de thrombine.

7. Procédé selon la revendication 6, comprenant en outre un stockage approprié de la composition pharmaceutique.

8. Utilisation *in vitro* de bivalirudine comme unique composant inhibiteur de thrombine pour la préparation et/ou le stockage de BMC appropriées pour la thérapie cellulaire.

9. Utilisation *in vitro* de bivalirudine comme substitut de l'héparine pour la préparation et/ou le stockage de BMC appropriées pour la thérapie cellulaire.
